Europäisches Patentamt

European Patent Office

Office européen des brevets

(1) Publication number: **0 042 253**
**B1**

(12) **EUROPEAN PATENT SPECIFICATION**

(45) Date of publication of patent specification: **09.10.85**

(51) Int. Cl.⁴: **A 61 L 15/04, D 21 H 5/12**

(21) Application number: **81302581.4**

(22) Date of filing: **10.06.81**

(54) Fibrous collagenous hemostatic-adhesive web and method for its preparation.

(30) Priority: **12.06.80 US 158734**

(43) Date of publication of application:
**23.12.81 Bulletin 81/51**

(45) Publication of the grant of the patent:
**09.10.85 Bulletin 85/41**

(84) Designated Contracting States:
**AT BE DE FR GB IT LU NL**

(56) References cited:
**US-A-3 810 473**
**US-A-3 823 212**
**US-A-4 016 877**
**US-A-4 066 083**
**US-A-4 089 333**
**US-A-4 148 664**

(73) Proprietor: **ALCON (PUERTO RICO) INC.**
**P.O. Box 3000**
**Humacao Puerto Rico 00661 (US)**

(72) Inventor: **Serravallo, Fred Albert**
**R.D. 2 Box 176B**
**Cranbury New Jersey 08512 (US)**

(74) Representative: **Warden, John Christopher et al**
**R.G.C. Jenkins & Co. 12-15, Fetter Lane**
**London EC4A 1PL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Description

This invention relates to non-woven sheets or webs particularly suited for medical and surgical purposes and consisting essentially of finely-divided fibers derived from natural collagen.

It has been known that collagen in various treated and prepared forms is useful in medical and surgical procedures and in the treatment of wounds. Collagen in certain forms has hemostatic properties when used as a wound dressing and has a low degree of antigenicity. In the U.S. Patent to Orlando A. Battista, Mamerto M. Cruz, Jr., and Merritt R. Hait, No. 3,742,955 granted July 3, 1973, there is described a fluffy mass of finely-divided fibers derived from native or natural collagen which when wet with blood has hemostatic properties and a unique adhesive property which is sufficient to join together severed biological surfaces.

As described in the aforementioned patent, the hemostat-adhesive material is in the form of a fluffy mass of finely-divided fibers. The fibrous mass has a bulk density of not more than about 0.128 g/ml (8 pounds per cubic foot), preferably the bulk density is between 0.024 and 0.96 g/ml (1.5 and 6.0 pounds per cubic foot). The fluffy mass when combined with blood in a wound forms a mass that is self-adherent to the tissue surfaces and will seal the wound without the use of sutures. The fibers may consist of collagen or a partial salt of collagen consisting essentially of an ionizable, water-insoluble, partial salt of collagen containing from about 50% to about 90% of the theoretical stoichiometric amount of the ionizable acid. The fluffy mass of finely-divided fibers for the use in the present invention may be prepared as described in the aforementioned patent.

Although the fluffy fibrous mass is highly efficaceous as a hemostat-adhesive material, the bulkiness and structureless nature of the product is disadvantageous from a handling standpoint and from the standpoint of delivering the product to a wound site. Because of the low density, structureless and fluffy nature of the product, it is necessary to transfer a mass of fibers to the wound site by the use of forceps or manually with rubber gloves. In such handling procedures, fibers become dislodged from the bulk being handled and portions of the mass of fibers adhere to the forceps or rubber gloves.

These fibers may be converted into sheets by conventional procedures used in the paper making art. However, because of the sensitivity to water of the collagen derived fibers, water-laid sheets or webs formed from these fibers are harsh and boardy and parchment-like in structure. Such parchment-like sheets do not conform readily to wound surfaces but most importantly exhibit a complete loss of hemostatic-adhesive properties.

U.S. Patent to Mamerto M. Cruz, Jr. Orlando A. Battista, LaVerne C. Tressler and Carmine Cirolla, No. 3,810,473 granted May 14, 1974, discloses a method of converting the fluffy mass of hemostatic-adhesive fibers into a liquid-laid web which retains the hemostatic and adhesive properties of the fibers. The production of harsh, parchment-like structures is avoided by slurrying the hemostat-adhesive fibers in a liquid consisting of a mixture having a composition, by volume, of from 95% organic liquid and 5% water to about 85% organic liquid and 15% water. When the organic liquid is ethanol, the mixtures contain, by weight, from 93.73% ethanol and 6.27% water to about 81.69% ethanol and 18.31% water. In the use of slurrying liquids containing more than 95% and up to 100% ethanol by weight, the products while receiving high "in vivo" ratings based upon hemostatic efficacy, adhesiveness to wound surfaces and delamination, the products were unsatisfactory from a handling standpoint. The cohesiveness was so poor that the sheets were crumbly, tore easily and were so delicate that they could not withstand normal shipping and handling.

In U.S. Patent to Mamerto M. Cruz, Jr., John H. Tenery and LaVerne C. Tressler, No. 4,016,877, granted April 12, 1977, there is disclosed a method of preparing liquid-laid, non-woven, fibrous webs which overcomes the disadvantages of the web disclosed in Patent No. 3,810,473. As disclosed in Patent No. 4,016,877, the fibers, as disclosed in Patent No. 3,742,955, are slurried in 100% ethanol containing a small proportion of concentrated hydrochloric acid, deposited to form a sheet or web, pressed under a limited pressure followed by freeze drying the sheet or webs. The only water contained in the slurrying liquid is that which is introduced by adding the hydrochloric acid, that which may be absorbed from the ambient atmosphere and that which is contained in the fibers. This added water amounts to from about 1.03% to about 5%.

These methods requiring the use of ethanol in the slurrying liquid are disadvantageous primarily because of the cost of the ethanol, about 3 liters of ethanol being required to produce a 15.24 cm×15.25 cm (6 inch by 6 inch) final trimmed sheet. Also, the open handling of large volumes of 100% ethanol presents certain hazardous conditions.

Although reference is made to ethanol as the organic liquid for the preparation of webs for medical and surgical purposes, for other purposes the ethanol may be replaced by other low molecular weight, water miscible organic solvents such as alcohols, ketones and the like, for example, methanol, isopropanol, amyl alcohol, methyl ethyl ketone, acetone and mixtures of these organic solvents. The use of the organic liquids other than ethanol is feasible. However, where the product is intended for surgical uses, it is essential that the product be completely freed of such other solvent. Moreover, the open handling of the large volumes of these other solvents also present hazardous conditions.

In U.S. Patent No. 3,157,524 granted November 17, 1964, to Charles Artandi, there is disclosed a method of preparing a collagen sponge. In accordance with this method, an acid dispersion of solvated collagen fibrils is frozen. The frozen structure is then immersed in a circulating bath consisting of a water miscible organic solvent such as isopropanol containing concentrated ammonium hydroxide. The organic solvent dehydrates the mass and the ammonium hydroxide neutralizes the acid. As indicated in an article entitled Use of Tanned Collagen Sponges in the Treatment of Liver Injuries by Erle E. Peacock, Hilliard F. Seigler and Paul W. Biggers, Annals of Surgery, Vol. 161, pp. 238—247, February, 1965, these collagen sponges do not possess an adhesive property. In use, it is essential that the sponge be mechanically maintained in contact with the wound surface.

In the U.S. Patent to Orlando A. Battista, No. 3,632,361, granted January 4, 1972, a method is disclosed whereby sponge-like bodies are prepared from ionizable, water-insoluble, partial salts of collagen of the type disclosed in Patent No. 3,742,955. In accordance with this method, the fibers consisting of the partial salt of collagen are attrited in water to form a stable dispersion. The stable dispersion is subsequently freeze dried to form a sponge-like body. The sponge-like body is substantially fiber-free and consists of film-like strata. The products lack both the hemostatic and adhesive characteristics of the partial salt fibers.

US—A—3823212 describes a process for producing felt-like membranes or sponge-like layers of collagen fibers which comprises decomposing skin and/or tendons or other animal connective tissues rich in collagen under acid and/or alkaline conditions, mechanically comminuting the decomposition product, suspending the communited collagen stock thus obtained in water to form a homogeneous slurry, adding a tanning or cross-linking agent, foaming the slurry and freezing it in the form of a layer at −5 to −40°C, incubating the frozen slurry layer for 1 to 30 days and then freeing the bulk of the water therefrom by mechanical squeezing and/or evaporative drying.

US—A—4066083 also discloses a process for producing freeze dried products from dispersions of collagen fibers. The products of the processes of US—A—3823212 and 4066083 do not however have any self-adherent properties, and when placed in position over a wound have to be held in place by an adhesive or bandages.

US—A—4148664 discloses a fluffy, finely-divided fibrous collagen derived product having hemostatic and adhesive properties sufficient to join together severed biological surfaces in a live warm blooded animal when the product is wet with blood between the surfaces. In the preparation of the product, water-wet collagen is treated with ethanol to remove water, is converted to an ionizable partial salt in the presence of ethanol and dehydrated with ethanol so as to control and limit the swelling of the collagen fibers and prevent hornification upon drying. The recovered dry material is subjected to deaggregation to form a mass having a bulk density of not more than 0.128 g/ml (8 pounds per cubic foot) and a surface area of at least 1 square meter per gram.

The present invention provides in one aspect a method of forming a velvet-like, hemostat-adhesive, fibrous, non-woven web dressing for severed biological surfaces the dressing being formed of hemostat-adhesive fibers consisting essentially of collagen or an ionizable, water-insoluble, partial salt of collagen containing from 50 to 90% of the theoretical stoichiometric amount of ionizable acid, the fibers having been subjected to deaggregation to impart self-adherent properties to the fibers, characterized by the steps of preparing a slurry of the collagen fibers, after deaggregation, in an aqueous liquid, the slurry containing from 0.5 to 3.5% fibers, by weight, introducing the slurry into a freeze-drier tray, in an amount sufficient to product webs having a thickness from 2 to 10.2 mm and a basis weight of 50 to 250 g . m$^{-2}$ and freeze drying the slurry to give a dressing which, when placed on a wound and wet with blood, self-adheres to the wound.

The method of the present invention overcomes the disadvantages of the use of the volatile organic solvent slurrying liquids.

In another aspect the invention provides a hemostat-adhesive dressing for severed biological surfaces characterized by a hemostat-adhesive fibrous, velvet-like, porous non-woven web consisting essentially of fibers of collagen or an ionizable, water-insoluble partial salt of collagen containing from 50 to 90% of the thereotical stoichiometric amount of ionizable acid and having a thickness of from 2 to 10.2 mm, a basis weight of from 25 to 250 g per sq.m. and a tensile strength of from 20 to 900 gms per 12.7 mm width, which is made by the method defined above and which, when placed on a wound and wet with blood, self-adheres to the wound.

The products of the present invention are fibrous, non-woven mats or webs which retain the hemostatic-adhesive properties of the fluffy mass of finely-divided fibers derived from collagen.

The present invention also provides, fibrous non-woven mats or webs which are flexible, non-flaking and possess sufficient cohesiveness to withstand normal handling in use and in shipping without separation of fibers.

The fibrous collagenous material can be prepared from any undenatured collagen in the natural state or delimed edible forms of collagen, such as, for example, hide, gut, tendon or other high fibrous collagen source material. Satisfactory raw materials for the collagen include fresh bovine hides, pigskin, sheepskin and the like such as used conventionally for the preparation of leather. Preferably the raw material consists of edible corium derived from fresh bovine hides.

In the preparation of the fibers, the collagen source material in wet state such as corium is diced or chopped into small chips or fragments of from about 6 to 12.7 mm (0.25 to about 0.5 inch) sizes in a cutting mill such as an Urschel Mill.

Alternatively, the chips may be fiberized as by mixing with crushed ice and the mixture passed through an Urschel Mill having cutting heads of smaller dimensions than those used in forming the chips. In processing of the collagen source material into a fluffy mass of fibers it is essential that the swelling or hydration of the fibers is controlled. If swelling or hydration of the fibers is excessive, many more sites are produced for hydrogen bonding thereby resulting in excessive hornification or densification upon drying. Such hornification may render the dried material extremely resistant to mechanical shredding or fiberization required to produce the required low bulk density and fluffiness to impart efficacious hemostatic and adhesive characteristics.

Accordingly, the collagen source material is processed in a liquid medium consisting of a major proportion of a water-miscible organic solvent such as a low molecular weight alcohol, preferably ethanol, with the balance water. For example, the collagen chips are first slurried in a liquid consisting of 70% ethanol and 30% (by weight) water including the water contained in the wet chips. After mixing has been conducted for a period sufficient to effect an equilibrium between the ethanol content of the chips and the liquid, the chips are separated from excess liquid and the recovered chips again slurried in an aqueous liquid containing a higher proportion of the organic solvent such as ethanol. In each successive slurrying operation the proportion of the organic solvent is increased so that after several slurrying operations the water content of the collagen has been reduced to about 1 to 2%. The collagen is separated from the liquid as by centrifugation and then dried. Drying may be effected either by oven drying or preferably by vacuum drying as at, for example, 60°C under a 94.7 KPa (29 inch) (710 mm . Hg) vacuum for about 16 hours. In general, such vacuum drying reduces the volatiles content to about 1%.

Where it is desired to produce an ionizable, water-insoluble, partial salt of collagen, the required amount of the ionizable acid may be added to the aqueous slurrying liquid. Preferably, the acid is incorporated in the second slurrying operation. The amount of acid added to the liquid is such as to form a partial acid salt with a bound acid content of from about 50 to 90%, preferably 60 to 85%, of the theoretical stoichiometric bound acid content. After the collagen has been slurried in the acid containing liquid for a period sufficient to form the partial salt, the collagenous material is separated from the liquid and subjected to slurrying operations as above described. Hydrochloric acid is the preferred acid for the preparation of the partial salt of collagen. Other ionizable inorganic and organic acids are satisfactory, such as, for example, sulfuric acid, hydrobromic acid, phosphoric acid, cyanoacetic acid, acetic acid, citric acid and lactic acid.

The dried collagenous material is converted into a fluffy mass of finely divided fibers by a deaggregating treatment. Prior to this treatment the material is preferably conditioned so as to contain between about 8 and 15% volatiles such as water and/or organic solvent. Conditioning may be effected by maintaining the dried collagenous material in an atmosphere at normal atmospheric temperatures and humidities (21° to 24°C, 40% to 60% R.H.) for from about 8 to 24 hours. Fiberization to the required bulk density and surface area may be accomplished by apparatus such as a hammer mill type comminution mill, for example, a Fitz Mill.

For the preparation of products pursuant to the present invention, fluffy masses of finely-divided, collagenous fibers were derived from wet or green bovine corium. The corium was cut into chips by the use of a high speed cutter (Urschel Mill). A portion of the chips was processed by the use of ethanol and a portion was processed by adding hydrochloric acid to the second ethanol slurrying liquid as described above. The amount of acid was sufficient to form a water-insoluble, ionizable, partial hydrogen chloride salt of collagen containing about 85% of the stoichiometric amount of hydrogen chloride.

The processed chips were vacuum dried at about 60°C at a pressure of about 23.3 KPa (175 mm . Hg) for about 6 hours followed by reducing the pressure to about 3.3 KPa (25 mm . Hg) and continuing the drying for about 6 hours. The dried chips were then conditioned by maintaining the chips in a clean room atmosphere at about 22°C, 50% R.H. for about 16 hours so as to increase the moisture content to about 10%. The conditioned chips were fiberized by passing them through a Fitz Mill (Model DA50-6-563) operated at 5000 rpm equipped with a special slotted screen having openings 1.6 mm×12.7 mm (0.062 in×0.5 in) with the slots at an angle of 30° to the sides of the screen. The fiberized material was subsequently passed through the Fitz Mill to produce the fluffy masses of finely-divided fibers.

Products were prepared by adding fibers to slurrying liquids consisting of distilled water and mixtures of distilled water and ethanol and the fibers slurried in the liquid by gently stirring the mass. The slurries contained from about 0.5% to about 4.0% fibers (dry basis). Each of the slurries was transferred to a freeze-dryer tray lined with a polyethylene film. The amount of slurry transferred to the tray was dependent upon the desired thickness and basis weight (gm./sq.m.) of the product. Freeze drying was effected in a Repp Freeze dryer, Model 40. The loaded tray was placed on the dry shelf having a temperature of about −40°C. A period of from about 1 to about 1.5 hours, depending upon the thickness and basis weight, was required to freeze the slurries. Vacuum was then applied, heating of the shelf was initiated to raise the temperature of the shelf to 38°—40°C and the absolute pressure reduced to about 6.7 CPa (50 microns Hg). The frozen slurries required from about 24 to about 48 hours to dry depending upon the thickness.

Products prepared as described were subjected to various physical testing as reported in Table I.

Certain products were also employed in surgical test procedures adapted to illustrate the efficacy of the products as a hemostat-adhesive material.

The Basis Weight and Thickness of the products are dependent upon the solids content of the slurry and the quantity of slurry added to the freeze dryer tray. As illustrated by Examples 6, 8 and 10, the thickness and basis weight of the products vary directly with the solids concentration of the slurry assuming the same quantity of the respective slurries is added to the freeze dryer tray.

The tensile strength of various products was measured on a standard Instron TM Tensile Tester, Model 1123, using a crosshead speed of 2.54 cm/min, a chart speed of 5.08 cm/min, a load range of 0.5 kg for Examples 9 and 11, a load range of 1 kg for Example 12 and a load range of 2 kg for Examples 6 and 7. In measuring the tensile strength, samples of the various Examples were cut to a width of 12.7 mm. The test was otherwise run according to the Technical Association for the Pulp and Paper Industry (TAPPI) Test Number T 414 ts 65. The results reported in Table I are the average breaking loads in grams for the 12.7 mm strips.

The porosity of samples was determined by the use of a mercury intrusion porosimeter, specifically an Aminco Winslow Mercury Porosimeter, Model 5-7121A. This type of instrument utilizes the principle that the volume of mercury penetrating certain size pores is directly related to the pressure supplied. A known weight of sample is placed in a calibrated tube, the system evacuated and then filled with mercury. Pressure is applied to the system in increments up to 3.45 MPa (5000 psi). The volume of mercury forced into the pores at various pressure readings is recorded. A plot of the pressure-volume relationship yields a graph from which pore diameter and penetration data can be read directly. The tests indicated that substantially all pores were less than 15 µm in diameter. The results reported in Table I specify that 75% of the pores have a diameter less than the stated value.

The stiffness of the webs was determined by maintaining samples wrapped on a glass cylinder for 24 hours at room temperature (22°C) and a relative humidity of about 50% and then releasing the samples and allowing them to flatten under their own weight. The samples were cut into 1.9 cm×7.9 cm strips, wrapped around a glass tube 2.54 cm in diameter and held in place by scotch tape. At the end of the 24 hour period, the tube was placed on a horizontal surface with the mid portion of the sample web in contact with the horizontal surface. The tape holding the ends of the tube together at the top of the tube was severed and the free ends of the web allowed to separate from the glass tube and uncurl under their own weight. The amount of uncurling was determined by measuring the angle between the horizontal surface and the uncurled ends of the sample. The angle in degrees is termed the Stiffness Factor as reported in Table I.

The stiffness factor is an indication of the ease with which the web may be conformed to the contour of the wound. The lower this factor the more readily it may be applied to conform to the contour. This factor may vary from 30 to 80, preferably from 35 to 65.

The water holding capacity of the webs was determined by accurately weighing 0.30 gram samples of webs and placing a sample on a 17.78 cm×17.78 cm (7 in×7 in) piece of cheesecloth. The corners of the cheesecloth were brought together and stapled to form a 'basket'. The basket was immersed in distilled water by means of forceps and maintained submerged in the water for 60 seconds. The wet basket was then withdrawn and the excess water allowed to drain without shaking for 60 seconds. The wet basket and contained web were immediately weighed. The same procedure was used to determine the water holding capacity of a like piece of cheesecloth and staple. From the measured weights, the water holding capacity of the webs was calculated and is reported in Table I as grams of water per gram of web.

The nature of the slurrying liquid, water or mixtures of water and ethanol, does not have a measurable effect on the properties of the web. Obviously, the elimination of ethanol avoids the hazards encountered in the handling of the solvent and reduces costs.

The concentration of the fibers in the slurry has the greatest influence on the characteristics of the webs. As shown by Reference Examples 1, 2 and 3, webs formed from slurries containing 4% fibers are harsh, stiff and boardy and are unsatisfactory. Both the hemostatic and adhesive properties are poor and the webs do not conform to irregular wound surfaces due to the stiffness. Slurries containing as low as 0.5% fibers produce satisfactory webs. In forming products from the lower concentration slurries, sufficient amounts are applied to the tray to form webs of a thickness of at least 2.5 mm. In using these low solids slurries it is necessary to balance the thickness versus the basis weight. As shown by Examples 8 and Reference Example 10, where the thickness is below about 2.5 mm and the basis weight is below about 50 g/sqm, the products are thin, rather fragile and exhibit a 'sticky' feel to the touch.

As illustrated by Reference Examples 2 and 3 prepared from 4% solids slurries and Example 11 prepared from a 0.5% solids slurry, the webs had a thickness of 5.1 mm. The webs of Reference Examples 2 and 3 were harsh, stiff and boardy while the web of Example 11 was soft but somewhat fragile. The webs of Reference Examples 2 and 3 had a basis weight of 280 g/sqm while the web of Example 11 had a basis weight of 80 g/sqm. The webs of Reference Examples 2 and 3 are unsatisfactory as hemostat-adhesive webs while the web of Example 11 is satisfactory.

Webs satisfactory for the purposes of this invention may be prepared from slurries having solids contents of from 0.5% to 3.5%, preferably from 0.5 to 2.5%, basis weights of from about 25 to about 250 g/sqm, preferably from 80 to 200 g/sqm, and thicknesses of from 2.0 to 10.2 mm, preferably 2.5 to 7 mm.

A very distinguishing characteristic of the products of the present invention is their physical nature and appearance. Whereas prior freeze dried products resemble sponge-like bodies exhibiting a somewhat rough, irregular, pock-marked surface, the present products have a soft, regular and white velvet-like surface. The products are flexible and may be wrapped around an ordinary pencil without rupturing.

Certain of the products prepared in the Examples were employed in surgical test procedures designed to evaluate not only the efficacy of the webs as hemostat-adhesive materials for severed biological surfaces in warm blooded animals when wet with blood, but also to assess the handling and delamination characteristics. Severed biological surfaces include tissue, cartilage, vessels, bone and other normal parts of warm blooded animals that may require mending or joining.

In the surgical procedure, the handling characteristics and delamination characteristics were noted. The handling characteristics involve the cohesiveness, non-friability flexibility, stiffness, ability to cut swatches from a sheet with scissors to form a clean cut and the ease with which a swatch may be conformed to a wound. The delamination property involves the ability to remove excess marginal portions of the web without overcoming the adhesiveness of the portion of the web covering the wound areas. In practice, a swatch of a size so as to overlap the wound several millimeters on all sides is cut from a sheet. Delamination is desirable so that following hemostasis the excess, that is, the material overlapping the wound, may be removed and only that portion sealing the wound remains.

'In vivo' surgical procedures were carried out on anaesthetized mongrel dogs. The spleen of the dog was exposed and excised wounds were made, the wounds measured about 20×10 mm with a depth of about 1 to 2 mm. Swatches were cut from sample webs as described above, the swatches being of a size so as to overlap the wound about 4 to 5 mm on all sides. The investigation was provided with coded web samples without a knowledge of the history of the samples so that all evaluation was 'blind'.

A wound was swabbed with a dry, surgical cotton gauze pad so as to provide a freely bleeding wound and a swatch immediately placed over the wound. The swatch was held in place by the application of pressure with a dry cotton gauze pad. Pressure was applied for 2 minutes and the gauze pad lifted to determine whether hemostasis had been effected.

Delamination was determined after the swatch had been on the wound for 20 to 25 minutes. In this determination, excess material was removed by grasping the overlapping edges of the swatch with forceps and lifting the free edges. The adhesion of the material to the wound surface and the ease of removal of the material in excess of that required to prevent rebleeding and sealing of the wound was noted.

The investigator's evaluation of webs is set forth in Table II. As noted in Table II, swatches were cut from webs as prepared in Examples 6, 9 and 12 and Reference Example 10. A swatch from the web of Example 6, identified as Example 6B, was tested after sterilization by heating in an air oven at 110°C for 2 hours followed by heating at 126°C for 20 hours. The investigator reported that all samples tested were satisfactory (S) from the standpoint of Hemostatic Efficacy, Adhesive Quality and Handling characteristics. The Delamination characteristic of samples of Examples 6 and 12 and Reference Example 10 was satisfactory. Samples of Examples 6B and 9 were reported as being unsatisfactory (U) because it was difficult to remove the excess portions of the swatches without also lifting the edges of the portions adhering to the wound and allowing a bleeding of the wound where the edge has been lifted. Reapplication of pressure along such area again effects hemostasis. Because the small amount of the web not removed was so minor, these samples along with Examples 6 and 12 and Reference Example 10 were judged to be Satisfactory (S) Overall.

The webs of the present invention when placed on a wound and wetted with blood form a mass that arrests bleeding and that is self-adherent to the wound tissue and seals the wound. When the web is applied to the wound it is only necessary to apply pressure for a period sufficient to effect hemostasis after which the pressure may be removed.

The products are prepared from aqueous slurries containing between 0.5 and about 3.5%, preferably between 0.5 and 2.5%, fibers, by weight, having hemostat-adhesive properties and freeze drying the slurry. The fibers consist of collagen or an ionizable, water-insoluble, partial salt of collagen having a bound acid content of from 50 to 90%, preferably from 60 to 85%, of the theoretical stoichiometric bound acid content.

The products are fibrous having a soft, regular and white velvet-like surface and are sufficiently pliable and flexible to allow the webs to be wrapped around a rod having a diameter of 6 mm (0.25 inch) without cracking or rupturing. The thickness may vary from 2 to 10 mm, preferably from 2.5 to 7 mm. The basis weight may be between 25 and 250 g per sqm, preferably between 80 and 200 g per sqm. The tensile strength per 12.7 mm width may vary from 20 to 900 grams, preferably from 30 to 450 grams. The structure of the fibrous webs is such that substantially all pores have diameters not exceeding about 15 µm and 75% of the pores have diameters not exceeding 10 to 11 µm. The webs have

a Stiffness Factor varying from 30 to 85, preferably between 35 and 75. Webs comprising collagen fibers have a water holding capacity of from 5 to 10 grams of water per gram of web while webs comprising the partial salt of collagen will exhibit water holding capacities of from 20 to 35, preferably 22 to 32, grams of water per gram of web.

TABLE I

| Example | 1 (Ref) | 2 (Ref) | 3 (Ref) | 4 | 5 | 6 | 7 | 8 | 9 | 10 (Ref) | 11 | 12 |
|---|---|---|---|---|---|---|---|---|---|---|---|---|
| % Solids | 4 | 4 | 4 | 3 | 2.5 | 2 | 2 | 1 | 1 | 0.5 | 0.5 | 2 |
| Slurry liquid | W | W/E* | W/E** | W | W | W | W | W | W | W | W | W |
| Thickness, mm | 7.6 | 5.1 | 5.1 | 3.8 | 3.8 | 4.3 | 10.2 | 2.5 | 6.8 | 1.3 | 5.1 | 6.4 |
| Basis weight, g/sqm | 325 | 280 | 280 | 120 | 115 | 105 | 230 | 50 | 115 | 25 | 80 | 185 |
| Tensile strength g/12.7 mm | | | | 181 | 149 | 429 | 894 | | 70.6 | | 30.2 | 224 |
| Porosity — 75% less than — µm | | | | 9.96 | 9.98 | 7.26 | 11.10 | | 10.30 | | 10.20 | 9.20 |
| Stiffness factor | | | | 80 | 79 | 52 | 61 | | 63 | | 38 | 61 |
| Water holding capacity g . $H_2O$/g sample | | | | 23.9 | 31.4 | 27.2 | 24.7 | | 26.4 | | 26.6 | 7.2 |
| Character | H | H | H | V | V | V | V | VT | VS | VT | VSF | V |

W — Distilled water
* — 90% Water/10% Ethanol
** — 80% Water/20% Ethanol
H — Harsh, stiff, boardy
V — Velvet-like, soft, flexible, strong
VS — Velvet-like, soft, flexible
VSF — Velvet-like, soft, fragile
VT — Velvet-like, thin, fragile, sticky

0 042 253

TABLE II

| Ex. | Hemostat efficacy | Adhesive | Handling | Delamination | Overall |
|---|---|---|---|---|---|
| 6 | S | S | S | S | S |
| 6B | S | S | S | U | S |
| 9 | S | S | S | U | S |
| 10 | S | S | S | S | S |
| 12 | S | S | S | S | S |

S — Satisfactory
U — Unsatisfactory

## Claims

1. A method of forming a velvet-like, hemostat-adhesive, fibrous, non-woven web dressing for severed biological surfaces the dressing being formed of hemostat-adhesive fibers consisting essentially of collagen or an ionizable, water-insoluble, partial salt of collagen containing from 50 to 90% of the theoretical stoichiometric amount of ionizable acid, the fibers having been subjected to deaggregation to impart self-adherent properties to the fibers, characterized by the steps of preparing a slurry of the collagen fibers, after deaggregation, in an aqueous liquid, the slurry containing from 0.5 to 3.5% fibers, by weight, introducing the slurry into a freeze-drier tray, in an amount sufficient to produce webs having a thickness from 2 to 10.2 mm and a basis weight of 50 to 250 g . m$^{-2}$ and freeze drying the slurry to give a dressing which, when placed on a wound and wet with blood, self-adheres to the wound.

2. The method as defined in claim 1 characterized in that the slurry medium is a mixture of water and ethanol.

3. The method as defined in claim 2, characterized in that the slurry contains from 0.5 to 2.5% fibers, by weight.

4. The method as defined in any preceding claim characterized in that the fibers consist essentially of collagen.

5. The method as defined in any preceding claim characterized in that the fibers consist essentially of an ionizable, water-insoluble, partial salt of collagen containing from 60 to 85% of the theoretical stoichiometric amount of ionizable acid.

6. The method as defined in claim 5, characterized in that the fibers consist essentially of an ionizable, water-insoluble, partial hydrogen chloride salt of collagen.

7. The method as defined in any preceding claim characterized in that the slurry is frozen at a temperature of −40°C and the frozen slurry dried at a temperature of 38—40°C under a pressure of 50 μm.

8. A hemostat-adhesive dressing for severed biological surfaces characterized by a fibrous, velvet-like, porous non-woven web consisting essentially of hemostat-adhesive fibers of collagen or an ionizable, water-insoluble partial salt of collagen containing from 50 to 90% of the theoretical stoichiometric amount of ionizable acid and having a thickness of from 2 to 10.2 mm, a basis weight of from 25 to 250 g per sqm and a tensile strength of from 20 to 900 gms per 12.7 mm width, which is formed by the method of claim 1 and which, when placed on a wound and wet with blood, self-adheres to the wound.

9. A hemostat-adhesive dressing as defined in claim 8 characterized in that the web has a stiffness factor of from 30 to 85, measured by maintaining 1.9 cm×7.9 cm strips wrapped round a glass tube 2.54 cm in diameter for 24 hours at room temperature (22°C) and a relative humidity of about 50%, releasing the strips, allowing them to uncurl under their own weight on a horizontal surface and measuring the angle between the horizontal surface and the uncurled ends of the strips.

10. A hemostat-adhesive dressing as defined in claim 8 or claim 9 characterized in that the web consists essentially of fibers of collagen and the web has a water holding capacity of 5 to 10 grams of water per gram of web, measured by weighing a 0.3 g sample, placing it in a basket of a 17.75 cm×17.75 cm piece of cheesecloth, immersing the basket containing the sample and an identical empty basket in distilled water for 60 seconds, allowing the baskets to drain without shaking, weighing the wet basket and calculating the water holding capacity of the sample from these measured weights.

11. A hemostat-adhesive dressing as defined in claim 8 or claim 9 characterized in that the web consists essentially of fibers of an ionizable, water-insoluble partial salt of collagen and the web has a water holding capacity, measured as defined in claim 10, of from 20 to 35 grams of water per gram of wet.

12. A hemostat-adhesive dressing as defined in claim 9 characterized in that the web consists essentially of hemostat-adhesive fibers of collagen or an ionizable, water-insoluble partial

9

salt of collagen containing from 60 to 85% of the theoretical stoichiometric amount of ionizable acid, the web having a thickness of from 2.5 to 7 mm, a basis weight of from 80 to 200 gms per sqm, a tensile strength of from 30 to 450 gms per 12.7 mm width, a stiffness factor, measured as defined in claim 9, of from 35 to 75 and a porosity such that 75% of the pores have diameters not exceeding 10 to 11 µm and all pores have diameters not exceeding 15 µm.

13. A hemostat-adhesive dressing as defined in claim 12 characterized in that the web consists essentially of fibers of an ionizable, water-insoluble partial hydrogen chloride salt of collagen containing from 60 to 85% of the theoretical stoichiometric amount of hydrogen chloride and the web has a water holding capacity, measured as defined in claim 10, of from 20 to 35 grams of water per gram of web.

## Patentansprüche

1. Verfahren zur Bildung eines blutstillenden, klebenden Verbands für durchtrennte biologische Oberflächen in Form eines samtartigen Faservlieses, wobei der Verband aus blutstillenden, klebenden Fasern gebildet wird, die im wesentlichen aus Kollagen oder aus einem ionisierbaren, wasserunlöslichen partiellen Salz von Kollagen, das 50 bis 90% der theoretischen stöchiometrischen Menge ionisierbarer Säure enthält, bestehen, wobei die Fasern einer Entaggregierung unterzogen worden sind, um den Fasern Selbstklebeeigenschaften zu verleihen, gekennzeichnet durch die Schritte der Herstellung einer Aufschlämmung der Kollagenfasern in einer wäßrigen Flüssigkeit nach der Entaggregierung, wobei die Aufschlämmung 0,5 bis 3,5 Gew.-% Fasern enthält, der Einführung der Aufschlämmung in eine Gefriertrocknerschale in einer Menge, die zur Herstellung von Faservliesen mit einer Dicke von 2 bis 10,2 mm und einem Gewicht je Flächeneinheit von 50 bis 250 g . m$^{-2}$ ausreicht, und der Gefriertrocknung der Aufschlämmung, um einen Verband zu erhalten, der, wenn er auf eine Wunde gelegt worden und von Blut durchnäßt ist, selbst an der Wunde anklebt.

2. Verfahren nach Anspruch 1, dadurch gekennzeichnet, daß das Aufschlämmungsmedium eine Mischung aus Wasser und Ethanol ist.

3. Verfahren nach Anspruch 2, dadurch gekennzeichnet, daß die Aufschlämmung 0,5 bis 2,5 Gew.-% Fasern enthält.

4. Verfahren nach einem der vorhergehenden Ansprüche, dadurch gekennzeichnet, daß die Fasern im wesentlichen aus Kollagen bestehen.

5. Verfahren nach einem der vorhergehenden Ansprüche, dadurch gekennzeichnet, daß die Fasern im wesentlichen aus einem ionisierbaren, wasserunlöslichen partiellen Salz von Kollagen, das 60 bis 85% der theoretischen stöchiometrischen Menge ionisierbarer Säure enthält, bestehen.

6. Verfahren nach Anspruch 5, dadurch gekennzeichnet, daß die Fasern im wesentlichen aus einem ionisierbaren, wasserunlöslichen partiellen Chlorwasserstoffsalz von Kollagen bestehen.

7. Verfahren nach einem der vorhergehenden Ansprüche, dadurch gekennzeichnet, daß die Aufschlämmung bei einer Temperatur von −40°C zum Gefrieren gebracht wird und die gefrorene Aufschlämmung bei einer Temperatur von 38 bis 40°C unter einem Druch von 50 µm getrocknet wird.

8. Blutstillender, klebender Verband für durchtrennte biologische Oberflächen, gekennzeichnet durch ein samtartiges, poröses Faservlies, das im wesentlich aus blutstillenden, klebenden Fasern aus Kollagen oder aus einem ionisierbaren, wasserunlöslichen partiellen Salz von Kollagen, das 50 bis 90% der theoretischen stöchiometrischen Menge ionisierbarer Säure enthält, besteht und eine Dicke von 2 bis 10,2 mm, ein Gewicht je Flächeneinheit von 25 bis 250 g . m$^{-2}$ und eine Zugfestigkeit von 20 bis 900 g pro 12,7 mm Breite hat, durch das Verfahren von Anspruch 1 gebildet wird und, wenn es auf eine Wunde gelegt worden und von Blut durchnäßt ist, selbst an der Wunde anklebt.

9. Blutstillender, klebender Verband nach Anspruch 8, dadurch gekennzeichnet, daß das Faservlies einen Steifigkeitsfaktor von 30 bis 85 hat, wobei der Steifigkeitsfaktor gemessen wird, indem Streifen (1,9 cm×7,9 cm), die um ein Glasrohr mit einem Durchmesser von 2,54 cm herumgewickelt sind, 24 h lang bei Raumtemperatur (22°C) und einer relativen Feuchte von etwa 50% gehalten werden, die Streifen abgenommen werden und die Streifen unter ihrem eigenen Gewicht auf einer waagerechten Oberfläche entrollen bzw. gerade werden gelassen werden und der Winkel zwischen der waagerechten Oberfläche und den entrollten bzw. gerade gewordenen Enden der Streifen gemessen wird.

10. Blutstillender, klebender Verband nach Anspruch 8 oder 9, dadurch gekennzeichnet, daß das Faservlies im wesentlichen aus Fasern aus Kollagen besteht und daß das Faservlies ein Wasserfassungsvermögen von 5 bis 10 g Wasser pro g Faservlies hat, wobei das Wasserfassungsvermögen gemessen wird, indem eine Probe von 0,3 g abgewogen und in einen Korb aus einem Stück Mull (17,75 cm×17,75 cm) hineingebracht wird, der Korb, der die Probe enthält, und ein identischer, leerer Korb 60 s lang in destilliertes Wasser eingetaucht werden, die Körbe abtropfen gelassen werden, ohne daß sie geschüttelt werden, die feuchten Körbe gewogen werden und das Wasserfassungsvermögen der Probe aus diesen gemessenen Gewichtswerten berechnet wird.

11. Blutstillender, klebender Verband nach Anspruch 8 oder 9, dadurch gekennzeichnet, daß das Faservlies im wesentlichen aus Fasern aus einem ionisierbaren, wasserunlöslichen partiellen Salz von Kollagen besteht und daß das Faservlies ein Wasserfassungsvermögen von 20 bis 35 g Wasser pro g Faservlies hat, wobei das Wasserfassungsvermögen in der in Anspruch 10 definierten Weise gemessen wird.

12. Blutstillender, klebender Verband nach Anspruch 9, dadurch gekennzeichnet, daß das Faservlies im wesentlichen aus blutstillenden, klebenden Fasern aus Kollagen oder aus einem ionisierbaren, wasserunlöslichen partiellen Salz von Kollagen, das 60 bis 85% der theoretischen stöchiometrischen Menge ionisierbarer Säure enthält, besteht, wobei das Faservlies eine Dicke von 2,5 bis 7 mm, ein Gewicht je Flächeneinheit von 80 bis 200 g . m$^{-2}$, eine Zugfestigkeit von 30 bis 450 g pro 12,7 mm Breite, einen in der in Anspruch 9 definierten Weise gemessenen Steifigkeitsfaktor von 35 bis 75 und eine derartige Porosität hat, daß 75% der Poren Durchmesser haben, die 10 bis 11 μm nicht überschreiten, und alle Poren Durchmesser haben, die 15 μm nicht überschreiten.

13. Blutstillender, klebender Verband nach Anspruch 12, dadurch gekennzeichnet, daß das Faservlies im wesentlichen aus Fasern aus einem ionisierbaren, wasserunlöslichen partiellen Chlorwasserstoffsalz von Kollagen besteht, das 60 bis 85% der theoretischen stöchiometrischen Menge von Chlorwasserstoff enthält, und daß das Faservlies ein Wasserfassungsvermögen von 20 bis 35 g Wasser pro g Faservlies hat, wobei das Wasserfassungsvermögen in der in Anspruch 10 definierten Weise gemessen wird.

**Revendications**

1. Procédé pour former un pansement en tissu non tissé, fibreux, hémostatique, adhérent et du type velours, pour des surfaces biologiques sectionnées, le pansement étant formé de fibres hémostatiques adhésives consistant essentiellement en collagène ou en un sel partiel ionisable insoluble dans l'eau de collagène contenant de 50 à 90% de la quantité stoechiométrique théorique d'acide ionisable, les fibres ayant été soumises à une désagrégation pour être dotées de propriétés d'auto-adhérence, caractérisé en ce qu'il comprend les étapes consistant à préparer une suspension de fibres de collagène, après désagrégation, dans un liquide aqueux, la suspension contenant de 0,5 à 3,5% en poids de fibres, à introduire la suspension dans un plateau de lyophilisation en une quantité suffisante pour former des tissus ayant de 2 à 10,2 mm d'épaisseur et un grammage de 50 à 250 g . m$^{-2}$ et à lyophiliser la suspension pour obtenir un pansement qui, quand il est placé sur une blessure et est imprégné de sang, adhère de lui-même à la blessure.

2. Procédé selon la revendication 1, caractérisé en ce que le milieu de suspension est un mélange d'eau et d'éthanol.

3. Procédé selon la revendication 2, caractérisé en ce que la suspension contient de 0,5 à 2,5% en poids de fibres.

4. Procédé selon l'une quelconque des revendications précédentes, caractérisé en ce que les fibres consistent essentiellement en collagène.

5. Procédé selon l'une quelconque des revendications précédentes, caractérisé en ce que les fibres consistent essentiellement en un sel partiel ionisable insoluble dans l'eau, de collagène contenant de 60 à 85% de la quantité stoechiométrique théorique d'acide ionisable.

6. Procédé selon la revendication 5, caractérisé en ce que les fibres consistent essentiellement en un chlorhydrate partiel ionisable insoluble dans l'eau, de collagène.

7. Procédé selon l'une quelconque des revendications précédentes, caractérisé en ce qu'on congèle la suspension à une température de −40°C et on sèche la suspension congelée à une température de 38 à 40°C sous une pression de 50 μm.

8. Pansement hémostatique adhésif pour surfaces biologiques présentant une blessure, caractérisé en ce qu'il comprend un tissu non tissé, poreux, fibreux et du type velours consistant essentiellement en fibres hémostatiques adhésives de collagène ou d'un sel partiel ionisable insoluble dans l'eau de collagène contenant de 50 à 90% de la quantité stoechiométrique théorique d'acide ionisable et ayant une épaisseur de 2 à 10,2 mm, un grammage de 25 à 250 g . m$^{-2}$ et une résistance à la traction de 20 à 900 g pour 12,7 mm de largeur, en ce qu'il est formé par le procédé selon la revendication 1 et en ce que lorsqu'il est placé sur une blessure et imprégné de sang, adhère de lui-même à la blessure.

9. Pansement hémostatique adhésif selon la revendication 8, caractérisé en ce que le tissu présente un facteur de rigidité de 30 à 85, ce facteur étant mesuré en maintenant des rubans de 1,9 cm×7,9 cm enroulés sur un tube de verre de 2,54 cm de diamètre pendant 24 heures à température ambiante (22°C) et à une humidité relative d'environ 50%, en libérant les rubans, en leur permettant de se dérouler par leur propre poids sur une surface horizontale et en mesurant l'angle formé entre la surface horizontale et les extrémités des rubans après déroulement.

10. Pansement hémostatique adhésif selon la revendication 8 ou 9, caractérisé en ce que le tissu consiste essentiellement en fibres de collagène et en ce que le tissu possède une capacité de rétention d'eau de 5 à 10 g d'eau par gramme de tissu, cette capacité étant mesurée par une technique consistant à peser un échantillon de 0,3 g, à le placer dans un panier fait d'un morceau de mousseline de 17,75 cm×17,75 cm, à plonger le panier contenant l'échantillon et un panier identique vide dans de l'eau distillée, pendant 60 secondes, à permettre aux paniers de s'égoutter sans les secouer, à peser les paniers humides et à calculer la capacité de rétention d'eau de l'échantillon à partir des poids ainsi mesurés.

11. Pansement hémostatique adhésif selon la revendication 8 ou 9, caractérisé en ce que le tissu consiste essentiellement en fibres d'un sel partiel ionisable insoluble dans l'eau de collagène et présente une capacité de rétention d'eau, mesurée comme indiqué dans la revendication 10, de 20 à 35 g d'eau par gramme de tissu.

12. Pansement hémostatique adhésif selon la revendication 9, caractérisé en ce que le tissue consiste essentiellement en fibres hémostatiques adhésives de collagène ou d'un sel partiel ionisable insoluble dans l'eau de collagène contenant de 60 à 85% de la quantité stoechiométrique théorique d'acide ionisable, le tissu ayant une épaisseur de 2,5 à 7 mm, un grammage de 80 à 200 g . m⁻², une résistance à la traction de 30 à 450 g pour 12,7 mm de largeur, un facteur de rigidité, mesuré comme indiqué dans la revendication 9, de 35 à 75 et une porosité telle que 75% des pores ont des diamètres ne dépassant pas 10 à 11 μm et tous les pores ont des diamètres ne dépassant pas 15 μm.

13. Pansement hémostatique adhésif selon la revendication 12, caractérisé en ce que le tissue consiste essentiellement en fibres de chlorhydrate partiel ionisable insoluble dans l'eau de collagène contenant 60 à 85% de la quantité stoechiométrique théorique d'acide chlorhydrique et en ce que le tissu présente une capacité de rétention d'eau, mesurée comme indiqué dans la revendication 10, de 20 à 35 g d'eau par gramme de tissu.